# EUROPEAN PATENT SPECIFICATION

(11) **EP 4 503 928 B1**
(45) Date of publication and mention of the grant of the patent: **09.12.2026**
(21) Application number: 23722810.1
(22) Date of filing: 06.04.2023
(51) Int. Cl.: A01N 1/125

(54) **CRYOPRESERVATION MEDIUM COMPRISING HYALURONIC ACID, USE THEREOF AND METHOD OF CRYOPRESERVATION**
HYALURONSÄURE ENTHALTENDES KRYOKONSERVIERUNGSMEDIUM, DESSEN VERWENDUNG UND VERFAHREN ZUR KRYOKONSERVIERUNG
MILIEU DE CRYOCONSERVATION COMPRENANT DE L'ACIDE HYALURONIQUE, SON UTILISATION ET MÉTHODE DE CRYOCONSERVATION

(30) Priority: 08.04.2022 CZ 20220148
(43) Date of publication of application: 12.02.2025
(73) Proprietor: Contipro a.s., 56102 Dolni Dobrouc (CZ)
(72) Inventor: NESPOROVA, Kristina, 56201 Usti nad Orlici (CZ); PRAT, Tomas, 60200 Brno (CZ); PILBAUEROVA, Nela, 26717 Morina (CZ); SUCHANEK, Jakub, 50303 Vrchovnice (CZ); VELEBNY, Vladimir, 56401 Zamberk (CZ)
(74) Representative: Lunzarová, Lucie
(86) International application number: PCT/CZ2023/050017
(87) International publication number: WO 2023/193839

(56) References cited:
- LEE TAE WOOK ET AL: "Enhanced Cellular Cryopreservation by Biopolymer-Associated Suppression of RhoA/ROCK Signaling Pathway", MATERIALS, vol. 14, no. 20, 14 October 2021 (2021-10-14), pages 6056, XP093056482, DOI: 10.3390/ma14206056
- LEE TAE WOOK ET AL: "SUPPORTING INFORMATION Enhanced cellular cryopreservation by biopolymer- associated suppression of RhoA/ROCK signaling pathway", 14 October 2021 (2021-10-14), XP093056508, Retrieved from the Internet <URL:https://www.mdpi.com/article/10.3390/ma14206056/s1> [retrieved on 20230621]
- PILBAUEROVA NELA ET AL: "Innovative Approach in the Cryogenic Freezing Medium for Mesenchymal Stem Cells", BIOMOLECULES, vol. 12, no. 5, 20 April 2022 (2022-04-20), pages 610 - 626, XP093056347, Retrieved from the Internet <URL:https://www.ncbi.nlm.nih.gov/pmc/articles/PMC9138570/pdf/biomolecules-12-00610.pdf> DOI: 10.3390/biom12050610

## Description

### Technical field

The invention relates to the composition, use and preparation of a freezing medium for the long-term preservation of stem cell lines by cryopreservation. The medium contains hyaluronic acid and/or its sodium salt, with weight average molecular weight in the range from 1,000,000 to 2,200,000 g/mol, at concentration of 0.08 to 0.2 % (v/w), and DMSO at concentration of 3 - 5 % (v/v).

### State of the Art

Cryopreservation is a common method for the long-term preservation of biological material for subsequent research or clinical use. Cryopreservation uses low temperatures (-80 °C to -196 °C) at which metabolic processes are suspended. The optimal process of cryopreservation is when cells show a high degree of survival, viability and preserved functionality after thawing. In particular, for cell cultures of stem cells used for therapeutic purposes, it is essential to maintain a high proliferation activity of cryopreserved cells, as well as their pluripotency and genomic stability.

However, the process of cryopreservation can have serious consequences on cell survival. For this reason, it is necessary to use cryoprotective agents (CPAs), substances that reduce cellular damage during cryopreservation, affect the membrane integrity and maintain the balance of ionic forces between intra- and extracellular space. The most commonly used cryoprotectant dimethyl sulfoxide (DMSO) has a very narrow interface of action between cytoprotection and cytotoxicity, and there are doubts about its biological inertness (e.g. effect thereof on gene expression or epigenetic processes).

The negative effect of DMSO on stem cells in vitro is dose-dependent, an effect on viability, morphology, cell adhesion and differentiation was already observed within the concentration range of 0.1-1 % (v/v) at room temperature (Pal et al., 2012; Tunger et al., 2018). Cryopreserved fetal liver progenitor cells had a reduced expression of NANOG, OCT and SOX2 stem genes, and bone marrow cells had an altered morphology and function (Borisov et al., 2014; Czysz et al., 2015). For these reasons, efforts are made to reduce DMSO exposure to the lowest possible level, to reduce DMSO concentration or to replace it with an alternative CPA (Awan et al., 2020).

However, most such alternative cryoprotectants do not occur in mammalian cells and therefore their use in cryopreservation requires a precise knowledge of biocompatibility. Not so with hyaluronan (hyaluronic acid - HA), which is the main and natural component of stem cells niche (the microenvironment surrounding the stem cells) (Nevi et al., 2017).

HA is a charged hydrophilic non-sulphated linear polysaccharide, glycosaminoglycan (GAG) composed of repetitive disaccharide subunits of (β, 1-4)-glucuronic acid (GlcUA) and (β, 1-3)-N-acetyl glucosamine (GlcNAc). The length of the polymer, i.e. the molecular weight, determines the physiological properties of HA, the binding to ECM and cell receptors (e.g. CD44), and so affects the cellular regulation cascade (Monslow et al., 2015).

The mechanism of HA cryoprotective effects is unknown. However, HA's high hydratation capacity causing an ice crystal growth slowdown and its very low cytotoxicity could be beneficial for its use in cryopreservation (Gurruchaga et al., 2018; Ujihira et al., 2010). The use of HA in cryopreservation is due to the physico-chemical properties of HA resulting primarily from the HA concentration-molecular weight ratio.

Currently, among other things, a solution is known according to the application EP1648227, which aims at the use of HA as a viscoelastic substance with a cytoprotective character for the transport and preservation of the cornea for transplantation. The application, however, describes that the cryoprotective properties are due to the addition of DMSO, not HA.

Also, the procedure of cryopreservation is already described in the patent EP2885969B1, which in certain cases mentions HA content in cryopreservation of stem cells. The patent suggests the possibility that HA is an appropriate component of the freezing medium, but does not mention what molecular weight (and possibly of which origin) is the most appropriate. This procedure also relies on the cryoprotective properties of other CPAs, including propylene glycol, sucrose, and possibly other sugars (e.g. ethylene glycol).

The patent CN110074096B discloses the composition of a serum-free medium containing DMSO, hydroxyethylated starch (HES), catechin, sodium tetraborate, 0.8% - 2% (w/v) hyaluronan and vitamin C. In this patent, the cryoprotective properties of HA are not directly demonstrated, e.g. the use of DMSO and HES is in long-term use and it is not clear from the patent what MW of HA was used and for what improvements of cryopreservation HA is responsible when using high DMSO concentration.

The application CN113661977A also mentioned the use of polyglutamic acid, HA and trehalose, however, the absence of innovation was claimed precisely because of the use of trehalose, the cryoprotective properties of which have long been known. It is mentioned that the use of trehalose in a cryomedium, as described in CN112806354A, could be inferred in routine testing. In this application, HA, trehalose, Dextran, glucose and HES are again used as cryoprotective agents. The effect of HA on the cryopreservation efficacy has not been determined separately in this application either, so it does not imply whether the efficacy is due to the other components of cryomedium.

The patent CN110839614B mentions hyaluronic acid, but for separating cells from each other and allowing cell migration, proliferation and avoidance of differentiation, rather than for cryopreservation itself.

The patent KR102274228B1 mentions the use of sulphated hyaluronic acid, but for comparison also the use of non-sulphated HA in the range from 300,000 to 500,000 g/mol and concentrations of 0.1-2 mg/mL (w/v). However, the patent targets the claims to sulphated HA and salts thereof.

*"*Enhanced Cellular Cryopreservation by Biopolymer-Associated Suppression of RhoA/ ROCK Signaling Pathway" in Materials 2021, 14, 6056 discloses cryopreservation medium for cryopreservation of stem cells wherein the HA used have a molecular weight of 100,000 g/mol and 5000 g/mol.

### Summary of the invention

Although the use of HA is mentioned in the scientific and patent literature, none of these solutions imply hMSC cryopreservation process based on HA cryoprotective properties increasing their application potential and the process of eliminating negative effects of DMSO in hMSC cryopreservation using HA.

The above problems are largely solved in this invention describing a cryopreservation medium based on the cryoprotective properties of hyaluronic acid.

The subject of this invention is the use of native hyaluronic acid dissolved in salts for cultivation as a cryopreservation solution for cryopreservation of stem cells, cell lines and tissues from living cells.

The component of the cryopreservation solution is native hyaluronic acid and its sodium salt having a weight average molecular weight in the range from 1,000,000 to 2,200,000 g/mol, preferably in the range from 1,000,000 to 1,750,000 g/mol, more preferably 1,500,000 g/mol.

The native hyaluronic acid and its sodium salt are used in this invention at concentration of 0.08 - 0.2 % (w/v), preferably of 0.1 to 0.2 % (w/v) and more preferably 0.1 % (w/v).

The term "hyaluronic acid" means all forms of hyaluronic acid, from the acidic form (native; HA-COOH) to the sodium salt of hyaluronic acid (HA-COONa).

The components for the dissolution of native hyaluronic acid are salts for cultivation, meaning standard media such as Dulbecco's Modified Eagle Medium (DMEM), modified Eagle medium in alpha modification (α-MEM), Roswell Park Memorial Institute medium 1640 (RPMI-1640) and Hanks' Balanced Salt Solution (HBSS), preferably RPMI-1640 and HBSS in modification without the addition of NaHCO₃. The remaining component of the mixture is water.

Cell cultures refer to aseptic cultures of eukaryotic cells of animals such as epithelial, nerve, epidermal cells, keratinocytes, haematopoietic cells, melanocytes, chondrocytes, B and T type immune cells, red blood cells, macrophages, monocytes, fibroblasts, muscle cells and stem cells, more specifically embryonic, mesenchymal and induced pluripotent stem cells.

This invention, the cryopreservation medium, is advantageous in that it does not have to contain a chemically undefined component of fetal bovine serum (FBS).

The cryopreservation medium is used sterile and the composition according to this invention allows terminal sterilization by moist heat.

The cryopreservation medium disclosed in this invention contains a reduced concentration of DMSO over the commonly used concentration, namely 3 - 5% versus 10% DMSO (v/v), more preferably 3% DMSO (v/v).

Furthermore, the invention relates to the use of the cryopreservation medium disclosed above for the cryopreservation of stem cells, cell lines and tissues from living cells and the method of cryopreservation of stem cells, cell lines and tissues from living cells, using this medium, where the cryopreservation medium according to the invention is first added to the cell cultures of stem cells, cell lines or tissues from living cells and then the mixture is slowly frozen, for example at a rate of 1 °C/min, for subsequent preservation at -80 °C to -196 °C.

The use of cryopreservation medium according to the present invention allows a reduction of the necessary DMSO concentration and thereby a reduction of the negative effect of DMSO on cryopreserved cultures.

The use of cryopreservation medium according to the present invention increases the proliferation capacity of cryopreserved stem cells and thus leads to a higher number of stem cells obtained by subsequent cultivation.

The use of the cryopreservation medium according to the present invention maintains the surface phenotype of stem cells and enhances the expression of the surface marker associated with proliferation and pluripotency.

Using the cryopreservation medium according to the present invention does not change the ability of stem cells to differentiate into different developmental cell lines.

This project was supported by the European Regional Development Fund - Project INBIO (No. CZ, 02,1,01/0,0/0,0/16_026/0008451).

### Brief description of the drawings

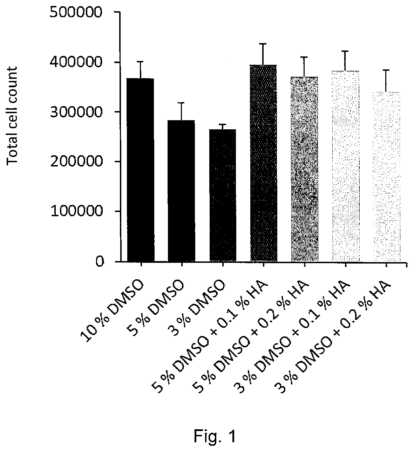

Fig. 1 represents the total number of hMSC cells measured using CaSy cell counter (OMNI Life Science GmbH) after two weeks of cultivation. The data are shown as averages and the standard deviation is shown as an error bar.

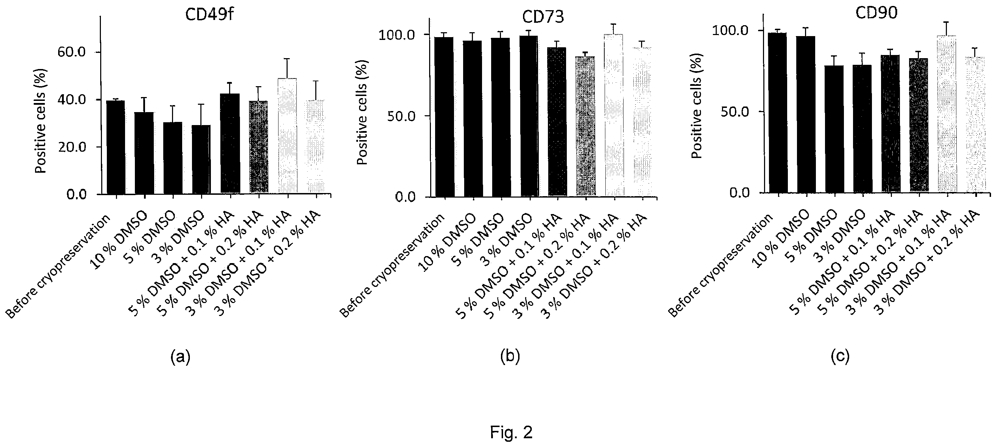

Fig. 2 represents the phenotypic profile of the hMSC surface receptors evaluated before cryopreservation and two weeks after thawing. The graph represents percentages of positive cells determined as a percentage with a fluorescent intensity greater than 99.5 % of negative isotype control. The data are shown as averages and the standard deviation is shown as an error bar.

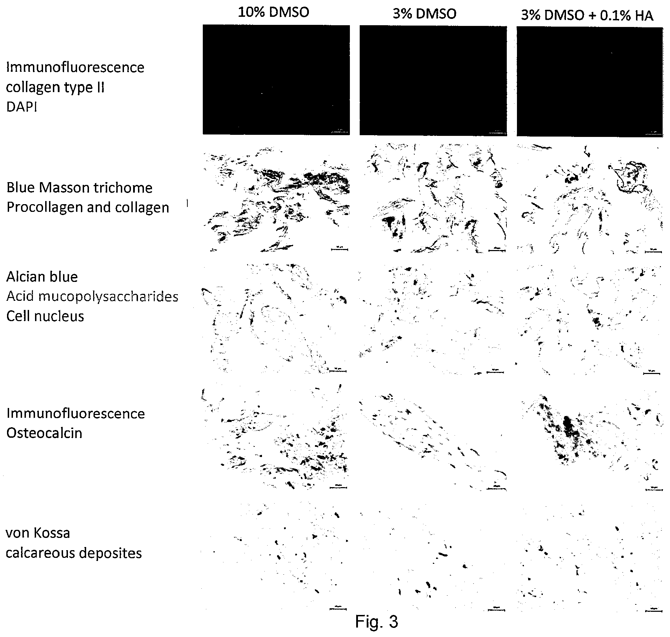

Fig. 3 represents immunocytochemical and histological staining to determine the differentiating potential of hMSC.

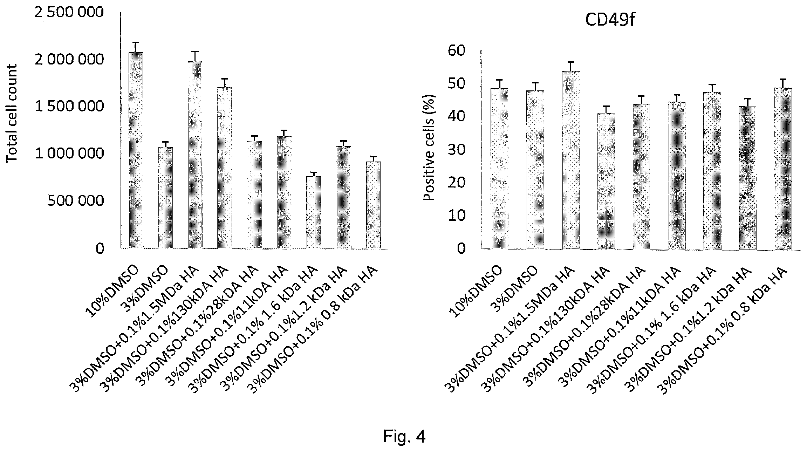

Fig. 4 on the left represents the total number of hMSC cells measured using CaSy cell counter (OMNI Life Science GmbH) after two weeks of cultivation. The data are shown as averages and the standard deviation is shown as an error bar. Fig. 4 on the right shows the phenotypic profile of the hMSC surface receptor evaluated two weeks after thawing. The graph represents percentages of positive cells determined as a percentage with a fluorescent intensity greater than 99.5 % of negative isotype control. The data are shown as averages and the standard deviation is shown as an error bar.

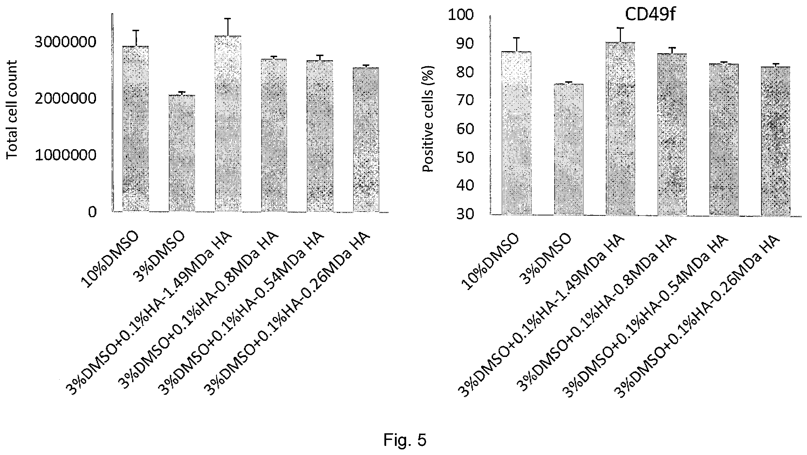

Fig. 5 on the left shows the total number of hMSC cells measured using CaSy cell counter (OMNI Life Science GmbH) after two weeks of cultivation. The data are shown as averages and the standard deviation is shown as an error bar. Fig. 5 on the right shows the phenotypic profile of the hMSC surface receptor evaluated two weeks after thawing. The graph represents percentages of positive cells determined as a percentage with a fluorescent intensity greater than 99.5 % of negative isotype control. The data are shown as averages and the standard deviation is shown as an error bar.

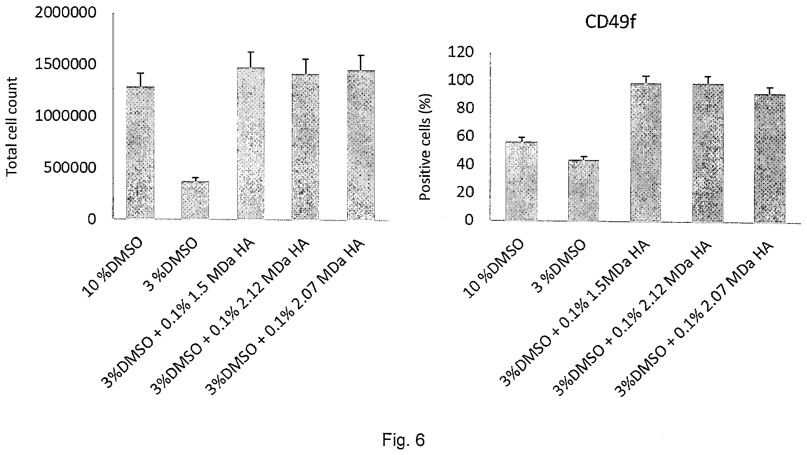

Fig. 6 on the left shows the total number of hMSC cells measured using CaSy cell counter (OMNI Life Science GmbH) after two weeks of cultivation. The data are shown as averages and the standard deviation is shown as an error bar. Fig. 6 on the right shows the phenotypic profile of the hMSC surface receptor evaluated two weeks after thawing. The graph represents percentages of positive cells determined as a percentage with a fluorescent intensity greater than 99.5 % of negative isotype control. The data are shown as averages and the standard deviation is shown as an error bar.

### Examples

### Example 1

Mesenchymal stem cell cultures were cryopreserved. The method of cryopreservation consists of the addition of a cryopreservation medium to the sample and the subsequent uncontrolled cryopreservation through a slow freeze at a rate of 1 °C/min, for preservation of the sample at low temperatures (-80 C to -196 C). The cryopreserved stem cell culture is revived after preservation by thawing in a water bath at 37 °C for 2 minutes and the cryopreservation medium is subsequently washed away from the cellular suspension by centrifugation. The cells are then seeded in a cultivation bottle and cultivated for two weeks.

For cryopreservation, 4 different combinations of cryoprotective medium containing hyaluronic acid with MW 1,500,000 g/mol in two concentrations of 0.1 and 0.2 % (w/v) with an addition of 5 or 3% DMSO (v/v) dissolved in a standard α-MEM medium were used. The composition containing 5, 3 or 10% (v/v) DMSO in the medium served as a cryopreservation control. DMSO concentration reduced to 5 - 3 % (v/v) resulted in lower MSCs survival and proliferation efficiency.

Surprisingly, the cryopreservation medium enriched with 0.1 or 0.2% (w/v) HA with MW 1,500,000 g/mol led to an increase in stem cell survival and proliferation efficiency. Contrary to expectations of a higher concentration of HA (0.2 % (w/v)) and a higher concentration of DMSO (5 % (v/v)) efficiency, the number of cryopreserved stem cells was highest in the combination of 3% DMSO (v/v) and 0.1% HA (w/v) with MW 1,500,000 g/mol, and after two weeks of cultivation exceeded the level of not only their control (3% DMSO (v/v)), but also 10% DMSO (v/v) (Fig. 1).

While the high expression of CD73 and CD90 markers was unaffected by the composition of the cryopreservation medium, we observed an increase in CD49f marker in cells cryopreserved using 3% DMSO and 0.1% HA with MW 1,500,000 g/mol, possibly associated with a higher proliferation activity (Fig. 2).

In the basic test of hMSC differentiation to the chondrogenic and osteogenic cell line, immunocytochemical staining did not reveal differences between cryomedia. Cells cryopreserved using 3% DMSO (v/v) and 0.1% HA (w/v) with MW 1,500,000 g/mol were able to differentiate into a chondrogenic and osteogenic cell line (Fig. 3).

The composition of the cryopreservation medium 3% DMSO (v/v) and 0.1% HA (w/v) with MW 1,500,000 g/mol is therefore effective for stem cell cryopreservation and is safe to maintain the key and unique properties of MSCs - high proliferation activity and pluripotency and thus allows the DMSO concentration to be reduced.

### Example 2

The mesenchymal stem cells hMSC cultures were cryopreserved. The method of cryopreservation consists of the addition of a cryopreservation medium to the sample and the subsequent uncontrolled cryopreservation for preservation of the sample at low temperatures (-80 °C to -196 °C). The cryopreserved stem cell culture is revived after preservation by thawing in a water bath at 37 °C for 2 minutes and the cryopreservation medium is subsequently washed away from the cellular suspension by centrifugation. The cells are then seeded in a cultivation bottle and cultivated.

Different molecular weights of hyaluronic acid in the range from 800 to 2,120,000 g/mol at concentration of 0.1 % (w/v) were used for stem cell cryopreservation, with an addition of 3% DMSO (v/v) dissolved in a standard medium for cells cultivation DMEM. To compare the efficacy, each formulation of cryopreservation medium was compared to a medium containing 10% and 3% DMSO (v/v) and 3% DMSO (v/v) together with 0.1% HA (w/v) with MW 1,500,000 g/mol.

Compared to the observed increase in the total number of hMSCs in cryopreservation medium with the combination of 3% DMSO (v/v) and 0.1% HA (w/v), no such increase over control with 3% DMSO (v/v) was observed for combinations with HA with MW in the range from 800 to 130,000 g/mL after two weeks of cultivation since thawing. The partial increase in the number of cells in the HA combination with MW 130,000 g/mL and 3% DMSO (v/v) was not surprisingly reflected in the expression of the surface marker CD49f compared to the combination of 3% DMSO (v/v) and 0.1% HA (w/v) with MW 1,500,000 g/mol (Fig. 4).

For combinations of cryopreservation medium with HA with MW in the range from 260,000 to 800,000 g/mL after two weeks of cultivation since thawing, an increase in the number of cells has already been observed, but surprisingly it did not reach the extent of the combination of cryopreservation medium 3% DMSO (v/v) and 0.1% HA (w/v) with MW 1,500,000 g/mol, although the increase in expression of surface marker CD49 was already evident (Fig. 5).

For combinations of the cryopreservation medium with HA with MW in the range from 2,070,000 to 2,120,000 g/mol after two weeks of cultivation since thawing, the increase in the number of cells in comparison with cryopreservation medium combination 3% DMSO (v/v) and 0.1% HA (w/v) with MW 1,500,000 g/mol was not different. Likewise, the increase in the expression of surface marker CD49 for these combinations of cryopreservation medium with HA is the same as for the combination of cryopreservation medium 3% DMSO (v/v) and 0.1% HA (w/v) with MW 1,500,000 g/mol (Fig. 6).

The composition of cryomedium 3% DMSOI0.1% HA is therefore effective for stem cell cryopreservation and is safe to maintain the key and unique properties of MSCs - high proliferation activity and pluripotency and thus allows the DMSO concentration to be reduced.

### Example 3

Mesenchymal stem cell cultures were cryopreserved. The method of cryopreservation consists of the addition of a cryopreservation medium to the sample and the subsequent uncontrolled cryopreservation for preservation of the sample at low temperatures (-80 C to -196 C). The cryopreserved stem cell culture is revived after preservation by thawing in a water bath at 37 °C for 2 minutes and the cryopreservation medium is subsequently washed away from the cellular suspension by centrifugation. The cells are then seeded in a cultivation bottle and cultivated for two weeks.

The cryoprotective medium containing hyaluronic acid with MW 1,500,000 g/mol at concentration of 0.08 and 0.1 % (w/v) was used for cryopreservation, with the addition of 5 or 3% DMSO (v/v) dissolved in a standard RPMI-1640 medium. The composition containing 3 or 10% (v/v) DMSO in the medium served as a cryopreservation control. The number of cryopreserved stem cells obtained after two weeks of cultivation, as well as the viability of cells and expression of markers CD49f, CD70 and CD90, were comparable to cryopreservation medium according to Example 1.

### Reference:

Awan, M., Buriak, I., Fleck, R., Fuller, B., Goltsev, A., Kerby, J., Lowdell, M., Mericka, P., Petrenko, A., Petrenko, Y., et al. (2020). Dimethyl sulfoxide: a central player since the dawn of cryobiology, is efficacy balanced by toxicity? Regen. Med. 15, 1463-1491.
Borisov, P.A., Dimitrov, A.Y., Ostankov, M.V., and Goltsev, A.N. (2014). Effect of Different DMSO Concentrations on Expression Level of Stemness Genes in Mice Fetal Liver Stem Cells Prior to and after Cryopreservation. Probl. Cryobiol. Cryomedicine 24, 185.
Czysz, K., Minger, S., and Thomas, N. (2015). DMSO efficiently down regulates pluripotency genes in human embryonic stem cells during definitive endoderm derivation and increases the proficiency of hepatic differentiation. PloS One 10, e0117689.
Gurruchaga, H., Saenz del Burgo, L., Orive, G., Hernandez, R.M., Ciriza, J., and Pedraz, J.L. (2018). Low molecular-weight hyaluronan as a cryoprotectant for the storage of microencapsulated cells. Int. J. Pharm. 548, 206-216.
Monslow, J., Govindaraju, P., and Puré, E. (2015). Hyaluronan - A Functional and Structural Sweet Spot in the Tissue Microenvironment. Front. Immunol. 6, 231.
Nevi, L., Cardinale, V., Carpino, G., Costantini, D., Di Matteo, S., Cantafora, A., Melandro, F., Brunelli, R., Bastianelli, C., Aliberti, C., et al. (2017). Cryopreservation protocol for human biliary tree stem/progenitors, hepatic and pancreatic precursors. Sci. Rep. 7, 6080.
Pal, R., Mamidi, M.K., Das, A.K., and Bhonde, R. (2012). Diverse effects of dimethyl sulfoxide (DMSO) on the differentiation potential of human embryonic stem cells. Arch. Toxicol. 86, 651-661.
Tunger, S., Gurbanov, R., Sheraj, I., Solel, E., Esenturk, O., and Banerjee, S. (2018). Low dose dimethyl sulfoxide driven gross molecular changes have the potential to interfere with various cellular processes. Sci. Rep. 8, 14828.
   Ujihira, M., Iwama, A., Aoki, M., Aoki, K., Omaki, S., Goto, E., and Mabuchi, K. (2010). Cryoprotective effect of low-molecular-weight hyaluronan on human dermal fibroblast monolayers. Cryo Letters 31, 101-111.

## Claims

1. A cryopreservation medium for cryopreservation of stem cells, cell lines and tissues from living cells, containing hyaluronic acid and/or a sodium salt thereof and DMSO, **characterised in that** the hyaluronic acid and the sodium salt thereof has a weight average molecular weight in the range from 1,000,000 to 2,200,000 g/mol and where hyaluronic acid and its sodium salt are present in the medium at concentration of 0.08 - 0.2 % (v/w), and DMSO is present in the medium at concentration of 3 - 5 % (v/v).

2. The cryopreservation medium according to claim 1, **characterised in that** the hyaluronic acid and the sodium salt thereof have a molecular weight in the range from 1,000,000 to 1,750,000 g/mol.

3. The cryopreservation medium according to claim 1, **characterised in that** the hyaluronic acid and the sodium salt thereof have a molecular weight of 1,500,000 g/mol.

4. The cryopreservation medium according to any of claims 1 to 3, **characterised in that** the hyaluronic acid and the sodium salt thereof are present at concentration of 0.1 % (v/w).

5. The cryopreservation medium according to any of claims 1 to 4, **characterised in that** the DMSO is present at concentration of 3 % (v/v).

6. The cryopreservation medium according to any of claims 1 to 5, **characterised in that** the hyaluronic acid and the sodium salt thereof are dissolved in a standard medium selected from the group comprising DMEM, MEM in alpha modification, RPMI-1640 and HBSS.

7. The cryopreservation medium according to any of claims 1 to 6, **characterised in that** the hyaluronic acid and the sodium salt thereof are dissolved in RPMI-1640 or HBSS in modification without the addition of NaHCO₃.

8. Use of the cryopreservation medium as defined in any of claims 1 to 7 for cryopreservation of stem cells, cell lines and tissues from living cells.

9. A method of cryopreservation of stem cells, cell lines and tissues from living cells, **characterised in that** it comprises the steps of:
a) addition of the cryopreservation medium as defined in any of claims 1 to 7 to stem cell cultures, cell lines or tissues from living cells,
b) slow freezing of cell cultures, cell lines or tissues in the cryopreservation medium from step (a) for subsequent preservation at -80 °C to -196 °C.

## Patentansprüche

1. Ein Kryokonservierungsmedium zur Kryokonservierung von Stammzellen, Zelllinien und Geweben aus lebenden Zellen, das Hyaluronsäure und/oder ein Natriumsalz davon sowie DMSO enthält, **dadurch gekennzeichnet, dass** die Hyaluronsäure und deren Natriumsalz ein gewichtsmittleres Molekulargewicht im Bereich von 1.000.000 bis 2.200.000 g/mol aufweisen und dass die Hyaluronsäure und deren Natriumsalz im Medium in einer Konzentration von 0,08 - 0,2 % (v/w) vorliegen, und DMSO im Medium in einer Konzentration von 3 - 5 % (v/v) vorliegt.

2. Das Kryokonservierungsmedium nach Anspruch 1, **dadurch gekennzeichnet, dass** die Hyaluronsäure und deren Natriumsalz ein Molekulargewicht im Bereich von 1.000.000 bis 1.750.000 g/mol aufweisen.

3. Kryokonservierungsmedium nach Anspruch 1, **dadurch gekennzeichnet, dass** die Hyaluronsäure und deren Natriumsalz ein Molekulargewicht von 1.500.000 g/mol aufweisen.

4. Kryokonservierungsmedium nach einem der Ansprüche 1 bis 3, **dadurch gekennzeichnet, dass** die Hyaluronsäure und deren Natriumsalz in einer Konzentration von 0,1 % (v/w) vorliegen.

5. Das Kryokonservierungsmedium nach einem der Ansprüche 1 bis 4, **dadurch gekennzeichnet, dass** das DMSO in einer Konzentration von 3 % (v/v) vorliegt.

6. Das Kryokonservierungsmedium gemäß einem der Ansprüche 1 bis 5, **dadurch gekennzeichnet, dass** die Hyaluronsäure und deren Natriumsalz in einem Standardmedium gelöst sind, das aus der Gruppe ausgewählt ist, die DMEM, MEM in der Alpha-Modifikation, RPMI-1640 und HBSS enthält.

7. Das Kryokonservierungsmedium gemäß einem der Ansprüche 1 bis 6, **dadurch gekennzeichnet, dass** die Hyaluronsäure und deren Natriumsalz in RPMI-1640 oder HBSS in einer Modifikation ohne Zugabe von NaHCO_{□}gelöst sind.

8. Verwendung des in einem der Ansprüche 1 bis 7 definierten Kryokonservierungsmediums zur Kryokonservierung von Stammzellen, Zelllinien und Geweben aus lebenden Zellen.

9. Verfahren zur Kryokonservierung von Stammzellen, Zelllinien und Geweben aus lebenden Zellen, **dadurch gekennzeichnet, dass** es die folgenden Schritte umfasst:
a) Zugabe des in einem der Ansprüche 1 bis 7 definierten Kryokonservierungsmediums zu Stammzellkulturen, Zelllinien oder Geweben aus lebenden Zellen,
b) langsames Einfrieren von Zellkulturen, Zelllinien oder Geweben in dem Kryokonservierungsmedium aus Schritt (a) zur anschließenden Lagerung bei -80 °C bis -196 °C.

## Revendications

1. Milieu de cryoconservation destiné à la cryoconservation de cellules souches, de lignées cellulaires et de tissus issus de cellules vivantes, contenant de l'acide hyaluronique et/ou un sel de sodium de celui-ci et du DMSO, **caractérisé en ce que** l'acide hyaluronique et son sel de sodium présentent un poids moléculaire moyen en poids compris entre 1 000 000 et 2 200 000 g/mol, et **en ce que** l'acide hyaluronique et son sel de sodium sont présents dans le milieu à une concentration de 0,08 à 0,2 % (v/p), et le DMSO est présent dans le milieu à une concentration comprise entre 3 et 5 % (v/v).

2. Milieu de cryoconservation selon la revendication 1, **caractérisé en ce que** l'acide hyaluronique et son sel de sodium ont un poids moléculaire compris entre 1 000 000 et 1 750 000 g/mol.

3. Milieu de cryoconservation selon la revendication 1, **caractérisé en ce que** l'acide hyaluronique et son sel de sodium ont un poids moléculaire de 1 500 000 g/mol.

4. Milieu de cryoconservation selon l'une quelconque des revendications 1 à 3, **caractérisé en ce que** l'acide hyaluronique et son sel de sodium sont présents à une concentration de 0,1 % (v/p).

5. Milieu de cryoconservation selon l'une quelconque des revendications 1 à 4, **caractérisé en ce que** le DMSO est présent à une concentration de 3 % (v/v).

6. Milieu de cryoconservation selon l'une quelconque des revendications 1 à 5, **caractérisé en ce que** l'acide hyaluronique et son sel de sodium sont dissous dans un milieu standard choisi parmi le groupe comprenant le DMEM, le MEM en modification alpha, le RPMI-1640 et le HBSS.

7. Milieu de cryoconservation selon l'une quelconque des revendications 1 à 6, **caractérisé en ce que** l'acide hyaluronique et son sel de sodium sont dissous dans du RPMI-1640 ou du HBSS dans une modification sans ajout de NaHCO₃.

8. Utilisation du milieu de cryoconservation tel que défini dans l'une quelconque des revendications 1 à 7 pour la cryoconservation de cellules souches, de lignées cellulaires et de tissus issus de cellules vivantes.

9. Procédé de cryoconservation de cellules souches, de lignées cellulaires et de tissus issus de cellules vivantes, **caractérisé en ce qu'**il comprend les étapes suivantes :
a) l'ajout du milieu de cryoconservation tel que défini dans l'une quelconque des revendications 1 à 7 à des cultures de cellules souches, des lignées cellulaires ou des tissus issus de cellules vivantes,
b) la congélation lente des cultures de cellules, des lignées cellulaires ou des tissus dans le milieu de cryoconservation de l'étape (a) en vue d'une conservation ultérieure à une température comprise entre -80 °C et -196 °C.
